(19) Europäisches Patentamt
European Patent Office
Office européen des brevets

(11) Veröffentlichungsnummer: **0 010 770**
**B1**

(12) **EUROPÄISCHE PATENTSCHRIFT**

(45) Veröffentlichungstag der Patentschrift: **07.07.82**

(51) Int. Cl.³: **C 07 D 213/75**, C 07 D 213/79, A 01 N 47/36

(21) Anmeldenummer: **79104284.9**

(22) Anmeldetag: **02.11.79**

# (54) In 2-Stellung substituierte 4-Pyridyl-harnstoffe bzw. -thioharnstoffe, Verfahren zu deren Herstellung sowie diese enthaltende Pflanzenwachstumsregulatoren.

(30) Priorität: **02.11.78 JP 135236/78**

(43) Veröffentlichungstag der Anmeldung:
**14.05.80 Patentblatt 80/10**

(45) Bekanntmachung des Hinweises auf die Patenterteilung:
**07.07.82 Patentblatt 82/27**

(84) Benannte Vertragsstaaten:
**CH DE FR GB IT NL**

(56) Entgegenhaltungen:
EP-A-0 001 979
GB-A-1 489 879
US-A-3 426 031
US-A-3 467 753
„Chemical Abstracts", Bd. 76, 1972, Zusammenfassung Nr. 113031y, S. 470, Columbus, Ohio, USA
T. LESIAK *et al.*: „New derivatives of urea"

**Die Akte enthält technische Angaben, die nach dem Eingang der Anmeldung eingereicht wurden und die nicht in dieser Patentschrift enthalten sind.**

(73) Patentinhaber: **Shudo, Koichi, Prof. Dr., 2000-10-2-116 Kosugayacho Totsukaku, Yokohama (JP)**
Patentinhaber: **Isogai, Yo, Prof. Dr., 1-1-2-609, Kamiyoga Setagayaku, Tokyo (JP)**
Patentinhaber: **Okamoto, Toshihiko, Prof. Dr., 1-7-19, Shinoharakita Kohokuku, Yokohama (JP)**
Patentinhaber: **Sato, Susumu, 20-7, Kamiyamacho Shibuya-ku, Tokyo (JP)**

(72) Erfinder: **Okamoto, Toshihiko, Prof. Dr., 1-7-19 Shinoharakita, Kohokuku, Yokohama (JP)**
Erfinder: **Shudo, Koichi, Prof. Dr., 2000-10-2-116 Kosugayacho, Totsukaku, Yokohama (JP)**
Erfinder: **Isogai, Yo, Prof. Dr., 1-1-2-609 Kamiyoga, Setagaya, Tokyo (JP)**
Erfinder: **Takahashi, Soshiro, 904-10, A-415, Oaza Kamiokubo, Urawashi, Saitama (JP)**

(74) Vertreter: **Wuesthoff, Franz, Dr.-Ing. et al, Patentanwälte Wuesthoff-v. Pechmann-Behrens-Goetz Schweigerstrasse 2, D-8000 München 90 (DE)**

EP 0 010 770 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

In 2-Stellung substituierte 4-Pyridylharnstoffe bzw. -thioharnstoffe, Verfahren zu deren Herstellung sowie diese enthaltende Pflanzenwachstumsregulatoren.

Die Erfindung betrift N-(2-substituierte-4-pyridyl)harnstoffe und -thioharnstoffe, Verfahren zu deren Herstellung sowie Wachstumsregulatoren für die Landwirtschaft, die als Wirkstoff diese Verbindungen enthalten.

Auf den Gebieten der Landwirtschaft und des Gartenbaus richtet sich das Augenmerk auf Pflanzenwachstumsregulatoren, die, wenn sie in Spuren angewandt werden, das Wachstum von Pflanzen beschleunigen oder unterdrücken können und die Pflanzen in den gewünschten Zustand bringen können.

Insbesondere wurden in letzter Zeit Verfahren zur Regelung des Pflanzenwachstums intensiv untersucht, bei denen die Hormonwirkung von chemischen Substanzen, wie Cytokinin oder Gibberellin ausgenutzt wird. Pflanzenwachstumsregulatoren, die eine hormonelle Aktivität wie Cytokinin besitzen (im folgenden als Cytokininhormonaktivität bezeichnet) können das Pflanzenwachstum beschleunigen, wenn sie in sehr geringen Mengen angewandt werden. Andererseits kann das Pflanzenwachstum manchmal unterdrückt werden, wenn derartige chemische Substanzen in einem Überschuss angewandt werden, d.h. in Mengen, die über diejenigen hinausgehen, die zur Beschleunigung des Pflanzenwachstums wirkt (manchmal als Überdosis bezeichnet).

Folglich bezeichnen die Ausdrücke „Pflanzenwachstumsregulation" und „Pflanzenwachstumsregulator" wie sie hier verwendet werden, in erster Linie eine Beschleunigung des Wachstums, manchmal jedoch auch eine Unterdrückung (bei einer Überdosierung) im weitesten Sinne. Derartige scheinbar entgegengesetzte Aktivitäten der Pflanzenregulatoren sind charakteristisch für die Cytokininaktivität. In dieser Beziehung sind keine Herbizide für die eine Cytokininaktivität klar festgestellt ist, bekannt, obwohl von einigen Herbiziden bekannt ist, dass sie Auxinaktivität besitzen. (Der Ausdruck „Auxin" ist ein allgemeiner Name für eine Art von Pflanzenwachstumsregulatoren).

Typische Pflanzenwachstumsregulatoren, von denen bekannt, ist, dass sie Cytokininhormonaktivität besitzen, sind 6-Benzyladenin, Kinetin und 4-Pyridylphenylharnstoff. In der EP-PS Nr. 78.101093 sind N-(2-Chlorpyridyl)harnstoffe und -thioharnstoffe angegeben. Es besteht jedoch nach wie vor Bedarf an einer Weiterentwicklung und Verbesserung von Pflanzenwachstumsregulatoren.

Aus „Chemical Abstracts", Bd. 76, 113031y (1972) sind bestimmte 1-Pyridyl-3-phenylharnstoffderivate bekannt, die als herbizide Mittel geeignet sind. Diese Verbindungen besitzen jedoch nur eine sehr geringe Wirkung als Pflanzenwachstumsregulatoren im oben angegebenen Sinne, d.h. insbesondere zur Beschleunigung des Wachstums.

In der US-PS Nr. 3467753 sowie der GB-PS Nr. 1489879 sind ebenfalls bestimmte Harnstoffderivate beschrieben. In diesen Druckschriften ist jedoch nur angegeben, dass diese Verbindungen als fungizide Mittel geeignet sind, bzw. als Zwischenprodukte zur Herstellung von Arzneimitteln für Menschen und Tiere auftreten. Auch diese Verbindungen sind als Pflanzenwachstumsregulatoren nicht geeignet.

Es ist Aufgabe der vorliegenden Erfindung, neue Pflanzenwachstumsregulatoren zu entwickeln, die mindestens gleich oder besser wirksam sind als die bekannten, sowie Mittel für die Landwirtschaft.

Diese Aufgabe wird gelöst durch N-(2-substituierte-4-Pyridyl)harnstoffe der allgemeinen Formel (I)

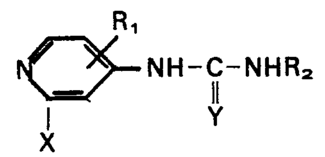
(I)

worin X ein Halogenatom, eine Hydroxy-, niedere Alkoxy-, niedere Alkylthio-, niedere Alkoxycarbonyl-, Amino-, niedere Alkylcarbonylamino-, Benzoylamino-, niedere Alkoxycarbonylamino-, Cyano- oder Trifluormethylgruppe, wobei die niedere Alkyl- oder Alkoxygruppe niedriger als $C_6$ sein soll, $R_1$ ein Wasserstoffatom, ein Halogenatom oder eine niedere Alkylgruppe, $R_2$ eine, gegebenenfalls durch eine niedere Alkyl- oder Alkoxygruppe, eine Hydroxygruppe, eine Trifluormethylgruppe oder ein Halogenatom substituierte, aromatische Gruppe und Y ein Sauerstoff- oder Schwefelatom bedeutet, wobei X nur dann ein Chloratom sein soll, wenn $R_1$ ein Halogenatom ist, sowie deren Säureadditionssalze.

Die erfindungsgemässen N-(2-substituierten-4-Pyridyl)harnstoffe und -thioharnstoffe der Formel (I) besitzen ausgezeichnete physiologische Wirksamkeiten gegenüber Pflanzen, die ähnlich den Wirkungen sind, die hervorgerufen werden durch Benzyladenin. Sie zeigen diese Wirkungen bereits, wenn sie in wesentlich geringerer Menge angewandt werden.

Die erfindungsgemässen Verbindungen können insbesondere das Pflanzenwachstum, die Zellmitose, die Zellvergrösserung, Zelldifferenzierung, Fruchtentwicklung, Blütenentwicklung oder Fruchtreifung beschleunigen oder sie können dazu dienen, dass diese Erscheinungen zu einem gewünschten Zeitpunkt eintreten. Sie können das Pflanzenwachstum auf ein gewünschtes Mass bringen, das Abfallen der Früchte verhindern, Kontrollmittel verhindern (z.B. Zucker, Alkaloide usw.), bei Früchten oder Zuckerrohr, den Geschmack von essbaren Pflanzen (Obst und Gemüse) verbessern usw.

Durch entsprechende Auswahl der Menge der erfindungsgemässen Verbindungen, üblicherweise in Form einer Überdosierung, können die erfindungsgemässen Verbindungen umgekehrt das Pflanzenwachstum auf einen gewünschten Grad unterdrücken, wobei eine sehr starke Unterdrückung des Pflanzenwachstums im allgemeinen als herbizide Wirkung bezeichnet wird.

So besitzen die erfindungsgemässen Pflanzenwachstumsregulatoren eine grosse Vielfalt von praktischen Eigenschaften, z.B. können sie nicht nur das Pflanzenwachstum fördern oder unterdrücken, sondern sie können auch eine Blüten- oder Fruchtbildung zu einem gewünschten Zeitpunkt sichern, die Bildung von samenlosen bzw. kernlosen Früchten ermöglichen, die Samen in einem „Schlaf"-Zustand halten oder aus einem „Schlaf"-Zustand „wecken", ein Abfallen der Blüten oder Früchte von Pflanzen und Bäumen verhindern und ein Abfallen der Blätter verhindern.

Bei den erfindungsgemässen Verbindungen der Formel (I) sind diejenigen besonders bevorzugt, bei denen X $OCH_3$, F, Br oder $CF_3$ oder X und $R_1$ jeweils Cl; Y, O oder S und $R_2$ eine durch F, Cl, $CF_3$ oder $CH_3$ substituierte Phenylgruppe ist. Besonders bevorzugt sind die folgenden Verbindungen:

N-(2-Trifluormethyl-4-pyridyl)-N'-(3-fluorphenyl)harnstoff,
N-(2-Fluor-4-pyridyl)-N'-phenylharnstoff,
N-(2-Methoxy-4-pyridyl)-N'-phenylharnstoff,
N-(2-Brom-4-pyridyl)-N'-phenylharnstoff,
N-(2-Trifluormethyl-4-pyridyl)-N'-phenylharnstoff,
N-(2-Methoxy-4-pyridyl)-N'-phenylthioharnstoff,
N-(2-Carbomethoxy-4-pyridyl)-N'-phenylharnstoff,
N-(2-Carbomethoxy-4-pyridyl)-N'-phenylthioharnstoff und
N-(2,6-Dichlor-4-pyridyl)-N'-phenylharnstoff.

Die erfindungsgemässen Verbindungen können nach den folgenden Reaktionen a, b oder c hergestellt werden:

a) Verbindungen der Formel II

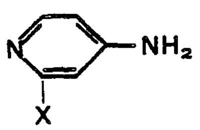 (II)

bei denen X die oben angegebene Bedeutung hat, werden umgesetzt mit Verbindungen der Formel III

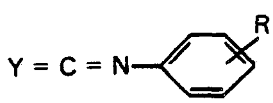 (III)

bei denen Y die oben angegebene Bedeutung hat, und R eine niedere Alkyl- oder Alkoxygruppe, eine Hydroxygruppe eine Trifluormethylgruppe oder ein Halogenatom bedeutet.

b) Verbindungen der Formel IV'

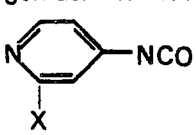 (IV')

oder

c) Verbindungen der Formel IV

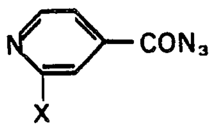 (IV)

bei denen X die oben angegebene Bedeutung hat (mit Ausnahme von $NH_2$ und OH) werden umgesetzt mit Verbindungen der Formel V

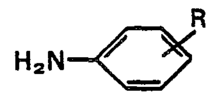 (V)

in denen R die oben angegebene Bedeutung hat.

An die oben angegebenen Reaktionen kann sich gegebenenfalls eine Hydrolyse, Acylierung oder Veresterung auf übliche Weise anschliessen, um einen Substituenten X in einen anderen Substituenten X umzuwandeln. Bei der obigen Reaktionsfolge sind die Verbindungen der Formel IV Vorstufen für die Verbindungen der Formel IV'.

Die oben angegebenen Umsetzungen a und b werden auf übliche Weise in Gegenwart oder Abwesenheit eines organischen Lösungsmittels bei Raumtemperatur oder unter Erhitzen durchgeführt.

Typische Beispiele für organische Lösungsmittel sind Benzol, Aceton, Pyridin usw., die besonders bevorzugt sind. Die Anwendung von Anilinen im Überschuss, die als Ausgangssubstanzen, wie oben angegeben, angewandt werden, ist vorteilhaft, da sie ebenfalls als Lösungsmittel dienen, wenn sie im Überschuss angewandt werden.

Während die Reaktionsbedingungen je nach dem herzustellenden Produkt variieren, werden im allgemeinen, soweit nicht anders angegeben, die folgenden Bedingungen angewandt:

Reaktion a):

Temperatur: unter Raumtemperatur bis ungefähr 100° C
Reaktionszeit: 10 min bis 30 h
Druck: üblicherweise Atmosphärendruck

Reaktion b):

Die Umwandlung von Verbindungen der Formel IV zu Verbindungen der Formel IV' erfordert üblicherweise eine Temperatur von ungefähr 50° C oder darüber, vorzugsweise 50 bis 150° C. Die Umsetzung von Verbindungen der Formel IV' mit den jeweiligen Anilinen wird üblicherweise bei Raumtemperatur bis 100° C durchgeführt. Folglich wird im allgemeinen ein Gemisch der Verbindungen der Formel IV und des Anilins bzw. der Aniline auf eine Temperatur von 50 bis 150° C ungefähr 30 min bis 10 h lang erhitzt. Wahlweise wird eine Lösung der gewünschten Verbindung(en) IV auf 50 bis 150° C ungefähr 30 min bis ungefähr 10 h erhitzt und dann die gewünschten Aniline zugegeben und nochmals auf eine Temperatur von 100° C erhitzt.

Die Hydrolyse, Acylierung oder Veresterung kann durchgeführt werden, um die Substituenten in den Reaktionsprodukten, wie sie durch die Umsetzungen a und b erhalten worden sind, in andere gewünschte Substituenten auf übliche Weise umzuwandeln. Die Reaktionsbedingungen für diese Hydrolyseacylierung und Veresterung sind im folgenden beispielhaft angegeben:

*Hydrolyse:*

Die Hydrolyse von z.B. Alkoxyverbindungen wird durchgeführt durch Erhitzen mit ungefähr 30%iger Jodwasserstoffsäure auf ungefähr 100° C in Essigsäure. Acetamidverbindungen werden hydrolysiert durch Erhitzen mit 6N-Salzsäure auf ungefähr 100° C.

*Acylierung:*

Die entsprechenden Amino- oder Hydroxyverbindungen werden mit Säureanhydriden oder Säurechloriden bei Raumtemperatur auf übliche Weise umgesetzt.

*Veresterung:*

Die Veresterung wird im allgemeinen durchgeführt durch Erhitzen mit Alkoholen auf 60 bis 100° C in Gegenwart einer Säure.

Von den Ausgangssubstanzen der Formeln II, III, IV, IV' und V sind die Verbindungen der Formel III und V im Handel erhältlich. Andere Verbindungen können auf übliche Weise hergestellt werden z.B. folgendermassen:

*Verbindung II*

Die entsprechenden 4-Nitroverbindungen oder 4-Nitroypridin-N-oxide werden in Gegenwart von Metallen wie Fe oder Zn oder katalytisch reduziert, um die Verbindungen der Formel II zu erhalten.

Wahlweise können Verbindungen der Formel II auch hergestellt werden durch Hydrolysieren der entsprechenden Azide oder Isocyanate.

*Verbindung IV*

Die entsprechenden Carboxyverbindungen werden in die entsprechenden Hydrazide umgewandelt und anschliessend mit Natriumnitrit umgesetzt.

*Verbindung IV'*

Verbindungen der Formel IV werden unter den oben angegebenen Verbingungen erhitzt.

Die erfindungsgemässen N-(2-substituierten-4-Pyridyl)harnstoffe und -thioharnstoffe besitzen hervorragende biologische Wirksamkeit auf Pflanzen, besonders eine ausgezeichnete Beschleunigungswirkung auf die Zellmitose, die Zellvergrösserung, die Zelldifferenzierung usw. und sind wirksam zur Beschleunigung der Fruchtbildung, Verhinderung des Abfallens von Früchten und Blüten, Wachstumsbeschleunigung, Gewichtszunahme von Blättern, Stengeln usw., Verzögerung des Alterns bzw. Welkens, Verhinderung von Frostschäden usw. Ferner tritt bei höherer Konzentration eine deutliche Unterdrückung des Wachstums (herbizide Wirksamkeit) auf und die erfindungsgemässen Verbindungen können z.B. in Herbiziden angewandt werden oder, um ein Pflanzenwachstum oder die Keimung zu verhindern.

Besonders bevorzugte Anwendungsgebiete sind die Beschleunigung der Fruchtbildung bei Kürbisgewächsen, wie Melonen, Wassermelonen usw.; eine Verhinderung des Abfallens von Blüten bei Weintrauben; eine Erhöhung des Gewichts von Stengeln und Töpfen (Spitzen, Trieben) bei Kidney-Bohnen usw.; Gewichtserhöhung von Blättern von *Datura sanguinea* und Tabakpflanzen; Verringerung der Pflanzenhöhe; Beschleunigung des Schösslingswachstums von Datura; Erhöhung der Grösse von Tabakkallus; Wachstum von Blättern aus Tabakkallus, Beschleunigung der Proliferation von gezüchteten Zellen; Verbesserung der Pflanzenbildung aus Kallus usw.

Die erfindungsgemässen Verbindungen können die Zellmitose wesentlich beschleunigen je nach ihrer Konzentration in dem für die Kultur der Pflanzenzellen angewandten Medium. Sie können angewandt werden in Kombination mit anderen Regulatoren, wie Auxin usw., wenn dies erwünscht ist, und können auch die Zelldifferenzierung von Stengeln und Blättern merklich beschleunigen.

Z.B. ist die optimale Konzentration zur Beschleunigung der Zellmitose von Tabakkallus in der folgenden Tabelle angegeben

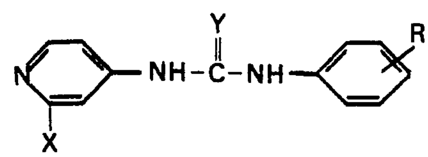

| Nr. | X | Y | R | Optimale Konzentration (ppm) | | |
|---|---|---|---|---|---|---|
| 1 | Br | O | H | 0,05 | — | 0,0005 |
| 2 | F | O | H | 0,005 | — | 0,0005 |
| 3 | $OCH_3$ | O | H | 0,1 | — | 0,001 |
| 4 | $NHCOCH_3$ | O | H | | — | 0,05 |
| 5 | $CF_3$ | O | H | 0,01 | — | 0,0005 |
| 6 | $CF_3$ | O | m-F | 0,001 | — | 0,0001 |

Wie aus der obigen Tabelle hervorgeht, sind die erfindungsgemässen Verbindungen gleich oder besser als 6-Benzyladenin, das eine der wirksamsten bekannten Cytokininverbindungen darstellt. Insbesondere besitzt die Verbindung 1 einen breiten Bereich für die optimale Konzentration. Das bedeutet eine leichte Handhabung der Substanz, d.h. es tritt auch bei verhältnismässig grober Dosierung des Mittels kein unbeabsichtigtes Welken der Pflanzen ein. Die Verbindung 3 führt immer zu einer ausgezeichneten Kallusbildung und die Verbindung 6 führt bei ausserordentlich geringer Dosierung zu guten Wirkungen.

Bezogen auf Untersuchungen an Tabakkallus-Sprösslingsbildung durch Zelldifferenzierung ist eine Menge, die ungefähr das 10 bis ungefähr

100fache der oben angegebenen beträgt, erforderlich.

Die Menge der erfindungsgemässen Verbindungen, wie sie zum direkten Besprühen von Pflanzen angewandt wird, beträgt im allgemeinen 10 bis 100 l/10 Ar in Form einer Lösung mit einer Konzentration von 0,0001 bis 10 000 ppm Wirkstoff, vorzugsweise 0,01 bis 10 000 ppm. Wenn die Verbindung auf den Boden aufgebracht wird, ist ungefähr die 5 bis 10fache Menge der oben angegebenen erforderlich. Selbstverständlich variiert die aufzubringende Menge je nach dem Ziel, das erreicht werden soll und der Pflanzenart, auf die die Verbindung aufgebracht wird.

Im allgemeinen werden 10 bis 100 l einer Lösung der folgenden Konzentration an erfindungsgemässer Verbindung oder Verbindungen als Wirkstoff pro 10 Ar angewandt.

| | | Art des Aufbringens |
|---|---|---|
| Zur Wachstumsbeschleunigung und Erhöhrung der Fruchtbildung | 0,01 bis 1000 ppm | direkt auf die Pflanzen z.B. durch Aufsprühen, Überziehen oder Eintauchen |
| Zur Wachstumsbeschleunigung (Proliferation oder Differenzierung von gezüchteten Zellen) | 0,0001 bis 100 ppm | Kulturmedium |
| Zur Beschleunigung des Abfallens der Früchte und Entlaubung | 0,1 bis 10 000 ppm | direkt auf die Pflanzen (wie oben angegeben) |
| Zur Wachstumsunterdrückung und als Herbizid | mehr als 10 bis 10 000 ppm | direkt auf Pflanzen (wie oben angegeben) oder auf den Boden |

Die erfindungsgemässen Verbindungen können allein oder im Gemisch mit anderen Substanzen oder Mitteln mit der gewünschten Wirkung angewandt werden, z.B. mit anderen Pflanzenregulatoren, Herbiziden, Insektiziden, Fungiziden und Acariziden; typischerweise in Form von Lösungen, Emulsionen, benetzbaren Pulvern, Körnern, Granulaten oder Pulvern.

Die Herstellung geeigneter Zubereitungen kann auf übliche Weise durchgeführt werden, z.B. durch Vermischen von 0,1 bis 50%, vorzugsweise 0,1 bis 10%, einer oder mehrerer erfindungsgemässen Verbindungen mit einem Träger (massebildenden Mittel) wie einem flüssigen oder festen Verdünnungsmittel oder Träger und soweit erwünscht oder notwendig einem Emulgier- oder Dispergiermittel.

Bevorzugte flüssige Verdünnungsmittel oder Träger umfassen Wasser, aromatische Kohlenwasserstoffe, wie Xylol, Benzol und Methylnaphthalin; chlorierte aromatische Kohlenwasserstoffe, wie Chlorbenzol, Mineralölfraktionen, wie Paraffin, Alkohole, wie Methanol und Propanol und polare Lösungsmittel, wie Dimethylformamid und Aceton.

Bevorzugte feste Verdünnungsmittel oder Träger sind u.a. Talkum, Ton, Kaolin, Kieselsäure, Holzpulver (Sägemehl) und Sand.

Bevorzugte Emulgiermittel umfassen Polyoxyäthylenfettsäureester oder Polyoxyäthylenfettsäurealkoholäther und bevorzugte Dispersionsmittel umfassen Alkylsulfonate, Alkylarylsulfonate, Alkalisalze, Erdalkalisalze, Ammoniumsalze von Ligninsulfonsäure und Methylcellulose.

Die erfindungsgemässen Verbindungen oder Mittel können als solche zu einem Medium gegeben werden oder sie können direkt auf die Pflanzen oder auf die Oberfläche der Blätter oder Stengel aufgebracht oder auf den Boden gesprüht werden. Sie werden im allgemeinen in Form eines üblichen Mittels bzw. einer Zubereitung angewandt. Ferner können die Pflanzenwachstumsregulatoren nach der Erfindung zusammen mit üblichen Düngemitteln und/oder Streckmitteln aufgebracht werden.

Die erfindungsgemässen Verbindungen können auch in Form eines anorganischen oder organischen Salzes, wie des Hydrochlorids, Phosphats, Sulfats, Citrats oder Tartrats angewandt werden.

Die Erfindung wird durch die folgenden nichteinschränkenden Beispiele näher erläutert.

Nachstehend werden zunächst anhand typischer Beispiele einige Herstellungsformen des Präparats vorgestellt. Die nachfolgenden Prozentangaben beziehen sich, sofern nichts anderes angegeben, jeweils auf das Gewicht.

*Rezept 1 Spritzpulver*

1% N-(2-Methoxy-4-pyridyl)-N'-phenylharnstoff
2% Natrium-β-naphthalinsulfonatformaldehydkondensat
2% Polyoxyäthylenalkylaryläther
95% Ton

*Rezept 2 Flüssigkeit*

1% N-(2-Fluor-4-pyridyl)-N'-phenylharnstoff
94% Dimethylformamid
5% Polyoxyäthylensorbitanmonolaurat

*Rezept 3 Lösung*

100 ppm N-(2-Methoxy-4-pyridyl)-N'-phenylharnstoff
20% Aceton
80% Wasser

Die folgenden Beispiele 1 bis 11 zeigen typische Verfahren zur Herstellung der erfindungsgemässen Verbindungen. Soweit nicht anders angegeben, erfolgte die Herstellung bei Zimmertemperatur und normalem Druck.

*Beispiel 1*

*Synthese von N-(2-Methoxy-4-pyridyl)-N'-phenylharnstoff*

Einer Lösung von 869 mg (7mmol) 2-Methoxy-4-aminopyridin in getrocknetem Benzol wurden 834 mg (7mmol) Phenylisocyanat beigefügt. Nachdem die Mischung bei Raumtemperatur 20 h lang gerührt wurde, setzten sich Kristalle ab, die abgefiltert und nach herkömmlicher Weise unter Verwendung von Aluminium chromatographiert wurden. Die Säule wurde mit Chloroform entwikkelt und das das Endprodukt enthaltende Eluat gesammelt. Das Lösungsmittel wurde unter vermindertem Druck abdestilliert und der Rückstand aus Benzol umkristallisiert, um 1,41 g N-(2-Methoxy-4-pyridyl)-N'-phenylharnstoff zu erhalten.

Ausbeute 82,9%

Schmelzpunkt 127 bis 129° C

Elementaranalyse für $C_{13}H_{13}N_3O_2$:

| | | | |
|---|---|---|---|
| Berechnet: | C 64,18 | H 5,39 | N 17,28% |
| Gefunden: | C 64,33 | H 5,33 | N 17,23% |

*Beispiel 2*

*Synthese von N-(2-Hydroxy-4-pyridyl)-N'-phenylharnstoff*

Zu 272 mg (11mmol) N-(2-Methoxy-4-pyridyl)-N'-phenylharnstoff wurden 2,5 ml Eisessig und 3,5 ml etwa 30%iger Hydrojodsäure hinzugefügt. Während des Rührens wurde die Mischung für 5½ h auf 95° C erhitzt. Nach Abkühlung wurde das Lösungsmittel abdestilliert. Zu dem sich ergebenden Rückstand, der dann mit Ammoniumcarbonat neutralisiert wurde, wurde eine geringe Menge Wasser beigefügt. Die sich absetzenden Kristalle wurden abgefiltert und aus Äthanol umkristallisiert, um 210 mg N-(2-Hydroxy-4-pyridyl)-N'-phenylharnstoff zu erhalten.

Ausbeute 82%

Schmelzpunkt 198 bis 200° C

Elementaranalyse für $C_{12}H_{11}N_3O_2 \cdot H_2O$:

| | | | |
|---|---|---|---|
| Berechnet: | C 58,29 | H 5,30 | N 17,00% |
| Gefunden: | C 58,20 | H 5,28 | N 16,94% |

*Beispiel 3*

*Synthese von N-(2-Acetamid-4-pyridyl)-N'-phenylharnstoff*

255 mg (1,7mmol) 2-Acetamid-4-aminopyridin wurden 35 ml getrocknetes Aceton beigegeben und die Mischung zur Auflösung des Aminopyridins erwärmt. Der sich daraus ergebenden Lösung wurden dann 201 mg (1,7mmol) Phenylisocyanat zugefügt und die Mischung unter Rückfluss 9 h lang gerührt. Nach Abkühlung wurde das Lösungsmittel abdestilliert und der Rückstand in herkömmlicher Weise unter Verwendung von Silicagel chromatographiert. Nach Entwicklung der Säule mit einer Chloroform/Aceton-(3:1 nach Volumen)-Mischung wurde das das Endprodukt enthaltende Eluat gesammelt. Nachdem das Lösungsmittel unter vermindertem Druck abdestilliert worden war, wurde der Rückstand aus einem Aceton/n-Hexan-Gemisch umkristallisiert, um 320 mg des Endprodukts zu erhalten.

Ausbeute 70,2%

Schmelzpunkt 188,5 bis 190 °C

Elementaranalyse für $C_{14}H_{14}N_4O_2$:

| | | | |
|---|---|---|---|
| Berechnet: | C 62,21 | H 5,22 | N 20,73% |
| Gefunden: | C 62,51 | H 5,25 | N 20,59% |

*Beispiel 4*

*Synthese von N-(2-Amino-4-pyridyl)-N'-phenylharnstoff*

173 mg (0,64mmol) N-(2-Acetamid-4-pyridyl)-N'-phenylharnstoff wurden 15 ml 6N-Salzsäure beigegeben. Die Mischung wurde danach in einem kochenden Wasserbad 6 h lang gerührt. Nach Abkühlung wurden die Kristalle, die sich abgesetzt hatten, abgefiltert. Ihnen wurde eine geringe Menge Wasser beigefügt, um sie unter Erwärmung aufzulösen. Um die Lösung zu neutralisieren wurde ihr danach eine wässrige Natriumcarbonatlösung beigegeben. Die sich absetzenden Kristalle wurden durch Abfilterung gesammelt und aus Methanol umkristallisiert, um 98 mg des Endprodukts zu erhalten.

Ausbeute 67,1%

Schmelzpunkt über 290 °C

Elementaranalyse für $C_{13}H_{12}N_4O$:

| | | | |
|---|---|---|---|
| Berechnet: | C 63,15 | H 5,30 | N 24,55% |
| Gefunden: | C 62,85 | H 5,27 | N 24,60% |

*Beispiel 5*

*Synthese von N-(2-Fluor-4-pyridyl)-N'-phenylharnstoff*

112 mg (1mmol) 2-Fluor-4-aminopyridin wurden 9 ml getrocknetem Benzol beigegeben. Der sich daraus ergebenden Lösung wurden 119 ml (1mmol) Phenylisocyanat beigefügt. Nach 30stündigem Rühren bei Raumtemperatur wurden die abgesetzten Kristalle durch Abfilterung gesammelt und danach in herkömmlicher Weise unter Verwendung von Aluminium chromatographiert. Nach Entwicklung der Säule mit einem Benzol/Äthylacetat-(3:1 nach Volumen)-Gemisch wurde das das Endprodukt enthaltende Eluat gesammelt. Nachdem das Lösungsmittel unter vermindertem Druck abdestilliert worden war, wurde der Rückstand aus einem Äthylacetat/n-hexan-Gemisch umkristallisiert, um 113 mg des Endproduktes zu erhalten.

Ausbeute 48,9%

Schmelzpunkt 179 bis 180° C

Elementaranalyse für $C_{12}H_{10}FN_3O$

Berechnet:

| | | | |
|---|---|---|---|
| C 62,33 | H 4,36 | N 18,17 | F 8,22% |

Gefunden:

| | | | |
|---|---|---|---|
| C 62,49 | H 4,36 | N 18,11 | F 8,15% |

*Beispiel 6*

*Synthese von N-(2-Brom-4-pyridyl)-N'-phenylharnstoff*

2-Brom-4-aminopyridin (433 mg; 2,5mmol) wurde 15 ml getrocknetem Benzol beigefügt und die Mischung zur Auflösung des Pyridins erwärmt. Der Lösung wurden 298 mg (2,5mmol) Phenylisocyanat beigefügt und die sich daraus ergebende Mischung 20 h lang bei Raumtemperatur gerührt. Die sich bildenden Kristalle wurden durch Abfilterung gesammelt und in herkömmlicher Weise unter Verwendung von Aluminium chromatographiert. Nach Entwicklung der Säule mit einem Benzol/Äthylacetat-(2,5:1 nach Volumen)-Gemisch wurde das das Endprodukt enthaltende Eluat gesammelt. Nachdem das Lösungsmittel unter vermindertem Druck abdestilliert war, wurde das Endprodukt aus 70%igem Methanol umkristallisiert, um 308 mg N-(2-Brom-4-pyridyl)-N'-phenylharnstoff zu erhalten.

Ausbeute 42,1%

Schmelzpunkt 188 bis 189° C

Elementaranalyse für $C_{12}H_{10}BrN_3O$:

| | | | |
|---|---|---|---|
| Berechnet: | C 49,34 | H 3,45 | N 14,38% |
| Gefunden: | C 49,40 | H 3,47 | N 14,10% |

*Beispiel 7*

*Synthese von N-(2-Trifluormethyl-4-pyridyl)-N'-phenylharnstoff*

Einer Lösung von 72 mg (0,33mmol) 2-Trifluormethylisonikotinoylazid in 5 ml getrocknetem Benzol wurden 31 mg (0,33mmol) Anilin zugegeben. Die sich daraus ergebende Mischung wurde unter Rückfluss 4 h lang gerührt. Nach Abkühlung wurde das Lösungsmittel unter verringertem Druck abdestilliert und der Rückstand in herkömmlicher Weise unter Verwendung von Silicagel (Lösungsmittel: $CHCl_3$: Aceton = 10:1 nach Volumen) chromatographiert. Die Fraktionen wurden gesammelt und das Lösungsmittel nochmals abdestilliert. Der Rückstand wurde aus Äthylacetat-n-hexan umkristallisiert, um 80 mg des gewünschten Produktes zu erhalten.

Ausbeute 85,1%

Schmelzpunkt 169 bis 171° C

IR-Spektrum (KBr):

3400 (−NH−) 1733 (−CO−)

Masse-Spektrum $M^+$ (m/e): 281

Elementaranalyse für $C_{13}H_{10}N_3F_3O$:

Berechnet:

C 55,52 H 3,58 N 14,94 F 20,27%

Gefunden:

C 55,58 H 3,57 N 14,96 F 20,34%

*Beispiel 8*

*Synthese von N-(2-Trifluormethyl-4-pyridyl)-N'-(3-Fluorphenyl)harnstoff*

In ähnlicher Weise wie bei Beispiel 7 wurden 101 mg von dem gewünschten Produkt gewonnen.

Ausbeute 91,0%

Schmelzpunkt 165 bis 167° C

IR-Spektrum (KBr): 1733 (−CO−)

Masse-Spektrum $M^+$ (m/e): 299

Elementaranalyse für $C_{13}H_9N_3F_4O$:

Berechnet:

C 52,18 H 3,03 N 14,04 F 25,40%

Gefunden:

C 52,12 H 2,96 N 13,76 F 24,92%

In analoger Weise kann unter Verwendung von 2,6-Dichlorisonikotinoylazid als Ausgangssubstanz N-(2,6-Dichlor-4-pyridyl)-N'-phenylharnstoff hergestellt werden.

Schmelzpunkt 219 bis 222° C

*Beispiel 9*

*Synthese von N-(2-Carbomethoxy-4-pyridyl)-N'-phenylharnstoff*

Einer Lösung von 186 mg (1,2mmol) Methyl 4-aminopicolat in 4 ml getrocknetem Aceton wurden 146 mg Phenylisocyanat beigefügt. Die sich daraus ergebende Mischung wurde über Nacht bei Raumtemperatur geschüttelt. Danach wurde das Lösungsmittel verdampft und der Rückstand in herkömmlicher Weise unter Verwendung von Silicagel (Lösungsmittel $CHCl_3$: Aceton = 4:1 nach Volumen) chromatographiert. Die Fraktionen wurden gesammelt und das Lösungsmittel abdestilliert. Der Rückstand wurde aus Methanol umkristallisiert, um 261 mg des gewünschten Produktes zu erhalten.

Ausbeute 78,6%

Schmelzpunkt 206 bis 208° C (druckentlastet)

IR-Spektrum (KBr): 1728,
1697 (−NHCONH−) und ($-COOCH_3$)

Masse-Spektrum $M^+$ (m/e):
kein Masse-Spektrum

Elementaranalyse für $C_{14}H_{13}N_3O_3$:

| | | | |
|---|---|---|---|
| Berechnet: | C 61,98 | H 4,83 | N 15,49% |
| Gefunden: | C 61,83 | H 4,85 | N 15,24% |

*Beispiel 10*

*Synthese von N-(2-Methoxy-4-pyridyl)-N'-phenylthioharnstoff*

Einer Lösung von 186 mg (1,5mmol) 2-Methoxy-4-aminopyridin in 15 ml getrocknetem Aceton wurden 203 mg (1,5mmol) Phenylisothiocyanat beigefügt. Die Mischung wurde dann einem 33stündigen Rückfluss unterzogen. Nach Abkühlung wurde das Lösungsmittel durch Destillation verdampft und der Rückstand in herkömmlicher Weise unter Verwendung von Silicagel (Lösungsmittel $CHCL_3$: Aceton = 10:1 nach Volumen) chromatographiert. Die Fraktionen wurden gesammelt und das Lösungsmittel abdestilliert. Der Rückstand wurde aus Äther umkristallisiert, um 130 mg des gewünschten Produktes zu erhalten.

Ausbeute 33,4%
Schmelzpunkt 138 bis 141 °C
Masse-Spektrum $M^+$ (m/e): 259
Elementaranalyse für $C_{13}H_{13}N_3OS$:

| | | | |
|---|---|---|---|
| Berechnet: | C 60,21 | H 5,05 | N 16,20% |
| Gefunden: | C 60,34 | H 5,07 | N 16,34% |

*Beispiel 11*

*Synthese von N-(2-Carbomethoxy-4-pyridyl)-N'-phenylthioharnstoff*

Einer Lösung von 187 mg (1,2mmol) Methyl-4-aminopicolat in 4 ml getrocknetem Aceton wurden 166 mg (1,2mmol) Phenylisothiocyanat beigegeben. Die Mischung wurde dann einem 30stündigen Rückfluss unterzogen. Nach Abkühlung wurde das Lösungsmittel abdestilliert und der Rückstand in herkömmlicher Weise unter Verwendung von Silicagel (Lösungsmittel: $CHCl_3$: Aceton = 8:1 nach Volumen) chromatographiert. Die Fraktionen wurden gesammelt und das Lösungsmittel abdestilliert. Der Rückstand wurde aus Äthylacetat umkristallisiert, um 141 mg des Endprodukts zu erhalten.

Ausbeute 39,9%
Schmelzpunkt 148 bis 149° C
IR-Spektrum (KBr): 1723 (−CO−)
Masse-Spektrum $M^+$ (m/e): 287
Elementaranalyse für $C_{14}H_{13}N_3O_2S$:

| | | | |
|---|---|---|---|
| Berechnet: | C 58,52 | H 4,56 | N 14,62% |
| Gefunden: | C 58,52 | H 4,65 | N 14,33% |

Die folgenden Beispiele veranschaulichen typische Anwendungsmöglichkeiten der erfindungsgemässen Verbindungen.

*Beispiel 12*

*Vermehrungstest-Wirkung von N-(2-Fluor-4-pyridyl)-N'-phenylharnstoff auf Tabakkalluszellen*

Tabakkallus wurde in Murashige-Skoog-Nährboden, welcher zwischen 0,0001 und 0,1 ppm N-(2-Fluor-4-pyridyl)-N'-phenylharnstoff und 2 ppm Indolessigsäure enthielt, 30 d lang bei 26 °C kultiviert. Das Endgewicht des frischen (nicht getrockneten) Kallus wird unten in Tabelle 1 angegeben. Zur Kontrolle wurde Tabakkallus in Murashige-Skoog-Nährboden kultiviert, der nur Indolessigsäure enthielt; die übrigen Bedingungen waren dieselben. Für Vergleichszwecke werden in Tabelle 1 auch die Werte angegeben, die in einem Nährboden, der die optimale Menge Benzyladenin enthielt, erzielt wurden. Die Werte sind jeweils Durchschnittswerte von 6 Proben. Die Messungen wurden in Übereinstimmung mit Torigoe und anderen, „Phytochemistry", Bd. 11, S. 1623 (1972) durchgeführt.

*Tabelle 1*

| Wirkstoff | Konzentration (ppm) | Rohgewicht (g) |
|---|---|---|
| Erfindungsgemässe Verbindung | 0,0001 | 1,9 |
| Erfindungsgemässe Verbindung | 0,001 | 5,5 |
| Erfindungsgemässe Verbindung | 0,01 | 4,5 |
| Erfindungsgemässe Verbindung | 0,1 | 1,2 |
| Benzyladenin | 0,01 | 5,4 |
| Benzyladenin | 0,001 | 1,0 |
| Kontrolle | — | 0,2 |

Es kann eindeutig festgestellt werden, dass die erfindungsgemässe Verbindung bei einer Konzentration von 0,001 ppm einen ähnlichen Ertrag brachte, wie er mit Benzyladenin bei einer Konzentration von 0,01 ppm erreicht wurde. Benzyladenin einer Konzentration von 0,001 ppm ergab einen wesentlich geringeren Produktionsertrag als die erfindungsgemässe Verbindung in gleicher Konzentration.

*Beispiel 13*

In gleicher Weise wie bei Beispiel 12 wurde der Vermehrungstest von Tabakkallus unter Verwendung von N-(2-Methoxy-4-pyridyl)-N'-phenylharnstoff durchgeführt. Die dabei erzielten Ergebnisse werden in Tabelle 2 dargelegt.

*Tabelle 2*

| Wirkstoff | Konzentration (ppm) | Rohgewicht (g) |
|---|---|---|
| Erfindungsgemässe Verbindung | 0,0001 | 0,5 |
| Erfindungsgemässe Verbindung | 0,001 | 2,1 |
| Erfindungsgemässe Verbindung | 0,01 | 6,7 |
| Erfindungsgemässe Verbindung | 0,1 | 2,7 |
| Benzyladenin | 0,01 | 5,7 |
| Kontrolle | — | 0,2 |

Die Ergebnisse zeigen, dass die optimale Konzentration der erfindungsgemässen Verbindung etwa dieselbe ist wie bei der Verwendung von Benzyladenin, jedoch ein besserer Ertrag erreicht wird.

*Beispiel 14*

*Sprossbildungstest-Wirkung von N-(2-Fluor-4-pyridyl)-N'-phenylharnstoff auf Markgewebe*

Einige Schnitte von Tabakmarkgewebe wurden in Murashige-Skoog-Nährboden, der 0,01 bis 10 ppm N-(2-Fluor-4-pyridyl)-N'-phenylharnstoff enthielt, geimpft. Der Nährboden enthielt kein Auxin. Nach 30tägiger Kultivierung bei Raumtemperatur wurde die Anzahl der sprossbildenden Markschnitte festgestellt. Die Ergebnisse sind in Tabelle 3 aufgeführt.

*Tabelle 3*

| Wirkstoff | Konzentration (ppm) | Sprossbildungsrate * |
|---|---|---|
| Erfindungsgemässe Verbindung | 10 | 12/12 |
| Erfindungsgemässe Verbindung | 1 | 12/12 |
| Erfindungsgemässe Verbindung | 0,1 | 7/12 |
| Erfindungsgemässe Verbindung | 0,01 | 0/12 |
| Benzyladenin | 1 | 7/12 |

* Anzahl der sprosstragenden Kallus/Gesamtzahl der Kallus

*Beispiel 15*

*Vergrösserung der Blattgrösse bei Frischgemüse mit N-(2-Methoxy-4-pyridyl)-N'-phenylharnstoff*

Aus einem Blatt *Brassica repa var. pervidis* wurden mit einem Korkbohrer runde Blattschnitte ausgeschnitten. Das Blatt liess man auf der Wasseroberfläche, das die oben bezeichnete Verbindung enthielt, treiben, um den Kontakt zwischen dem Wasser und der Rückseite der Blattschnitte sicherzustellen. Nach achttätigem Stehenlassen bei Raumtemperatur wurde der Durchmesser und das Gewicht der Blattschnitte bestimmt. Die Ergebnisse zeigt Tabelle 4. Die Werte sind Durchschnittswerte aus der Messung von je 10 Blattschnitten. Bei dem Kontrollversuch wurde reines Wasser verwendet.

*Tabelle 4*

| Konzentration | Durchmesser (mm) | Gewicht (mg) |
|---|---|---|
| 10 | 15,2 | 30,8 |
| 1 | 15,3 | 32,0 |
| 0,1 | 16,8 | 34,0 |
| 0,01 | 17,2 | 36,0 |
| 0,001 | 14,8 | 30,3 |
| Kontrolle | 12,8 | 27,5 |

Wie aus den Ergebnissen in der Tabelle 4 klar zu ersehen ist, beträgt die optimale Konzentration der erfindungsgemässen Verbindung 0,01 ppm. Im Vergleich zum Kontrollversuch waren sowohl Durchmesser als auch Gewicht der Blattschnitte um 30% grösser.

*Beispiel 16*

*Wirkung von N-(2-Methoxy-4-pyridyl)-N'-phenylharnstoff auf Gurkensprösslinge*

In Töpfen mit 10 cm Durchmesser wurden Gurkenpflanzen herangezogen. Als sie 3 bis 4 Blätter ausgetrieben hatten, wurden sie mit einer wässrigen Lösung, die 10 bis 100 ppm der oben bezeichneten Verbindung enthielt, besprüht. 15 d nach der Behandlung wurden Höhe, Stieldurchmesser und Gewicht des aus der Erde herausragenden Teils der Pflanze (Stiel und Blätter) gemessen. Die Ergebnisse werden in Tabelle 5 gezeigt. Höhe und Stieldurchmesser der Pflanzen sowie ihr Gewicht über dem Boden werden in Prozentzahlen angegeben, als Bezugswert (100%) dienen jeweils die entsprechenden Werte bei unbehandelten Pflanzen. Die Werte sind Mittelwerte von 4 Pflanzen.

*Tabelle 5*

| Konzentration der erfindungsgemässen Verbindung (ppm) | 10 | 50 | 100 |
|---|---|---|---|
| Höhe (%) | 95 | 91 | 91 |
| Durchmesser (%) | 100 | 110 | 118 |
| Gewicht (%) | 97 | 108 | 120 |

Wie die obigen Ergebnisse klar beweisen, konnte einerseits der Stieldurchmesser vergrössert werden, andererseits gelang es, die Pflanzenhöhe im Vergleich zum Kontrollversuch zu verringern. Dies bedeutet, dass durch die erfindungsgemässe Verbindung die Aufzucht von Pflanzen möglich ist, die starkem Wind und Kälte widerstehen können.

*Beispiel 17*

*Wirkung von N-(2-Brom-4-pyridyl)-N'-phenylharnstoff zur Verhinderung des Blütenabfalls bei Weintrauben*

Der Versuch wurde an einem im Freien gepflanzten Deleware-Weinstock vorgenommen. Die Blütenstände des Weinstocks wurden zur günstigsten Zeit für die Gibberellinbehandlung in eine wässrige Lösung von 100 ppm Gibberellin und 10 ppm

und 100 ppm der oben bezeichneten erfindungsgemässen Verbindung getaucht. 10 d nach der Vollblüte wurden alle Blütenstände nochmals getränkt und mit einer wässrigen Lösung, die nun lediglich 100 ppm Gibberellin enthielt, behandelt. Die Trauben wurden 53 d später geerntet und die Wirkung der Massnahmen zur Verhinderung des Blütenabfalls gemessen. Zum Vergleich werden in Tabelle 6 auch die Werte gezeigt, die bei einer Verwendung von 100 ppm Benzyladenin anstatt der oben genannten erfindungsgemässen Verbindung erzielt wurden.

*Tabelle 6*

| | Gibberellin (ohne Zusatz) | Erfindungsgemässe Verbindung 10 ppm | Erfindungsgemässe Verbindung 100 ppm | Benzyladenin 100 ppm |
|---|---|---|---|---|
| Gewicht der Fruchtstände (g) | 115 | 185 | 183 | 150 |
| Anzahl der Früchte | 90 | 136 | 140 | 118 |

Wie sich aus den obigen Resultaten klar ersehen lässt, wurde der Ertrag durch die erfindungsgemässe Verbindung deutlich verbessert.

*Beispiel 18*

*Versuch zur Gewichtssteigerung und Hemmung des Höhenwachstums bei* Datura sanguinea *durch Verwendung von N-(2-Methoxy-4-pyridyl)-N'-phenylharnstoff*

*Datura-sanguinea*-Pflanzen (Durchschnittshöhe 8 cm) wurden ins Freie umgesetzt. Als die Durchschnittshöhe der Pflanzen 20 cm erreicht hatte, wurde jede Pflanze mit 15 ml einer wässrigen Lösung der obengenannten erfindungsgemässen Verbindung in den in Tabelle 7 genannten Konzentrationen besprüht. Drei Wochen später wurden die Pflanzen abgeerntet und die Pflanzenhöhe sowie ihr Gesamtgewicht über dem Boden (Stamm und Blätter) gemessen. Die Ergebnisse zeigt Tabelle 7; die dort angegebenen Werte sind Durchschnittswerte von 5 Pflanzen.

*Tabelle 7*

| Wirkstoff | Konzentration (ppm) | Höhe (cm) | Gesamtgewicht (g) |
|---|---|---|---|
| Erfindungsgemässe Verbindung | 100 | 91 | 385 |
| Erfindungsgemässe Verbindung | 200 | 89 | 391 |
| Benzyladenin | 500 | 98 | 330 |
| Kontrollversuch | — | 99 | 303 |

Wie diese Resultate zeigen, vergrösserte sich der Ernteertrag im Vergleich zum Ertrag bei der Verwendung von Benzyladenin um etwa 30%.

**Patentansprüche**

1. N-(2-substituierte-4-Pyridyl)harnstoffe der allgemeinen Formel (I)

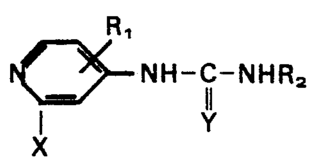

(I)

worin

X ein Halogenatom, eine Hydroxy-, niedere Alkoxy-, niedere Alkylthio-, niedere Alkoxycarbonyl-, Amino-, niedere Alkylcarbonylamino-, Benzoylamino-, niedere Alkoxycarbonylamino-, Cyano- oder Trifluormethylgruppe, wobei die niedere Alkyl- oder Alkoxygruppe niedriger als $C_6$ sein soll,

$R_1$ ein Wasserstoffatom, ein Halogenatom oder eine niedere Alkylgruppe,

$R_2$ eine, gegebenenfalls durch eine niedere Alkyl- oder Alkoxygruppe, eine Hydroxygruppe, eine Trifluormethylgruppe oder ein Halogenatom substituierte, aromatische Gruppe, und

Y ein Sauerstoff- oder Schwefelatom bedeutet, wobei X nur dann ein Chloratom sein soll, wenn $R_1$ ein Halogenatom ist,

sowie deren Säureadditionssalze.

2. N-(2-substituierte-4-Pyridyl)harnstoffe, nach Anspruch 1, dadurch gekennzeichnet, dass $R_1$ ein Wasserstoffatom bedeutet, sowie deren Säureadditionssalze.

3. N-(2-substituierte-4-Pyridyl)harnstoffe, nach Anspruch 1, dadurch gekennzeichnet, dass Y ein Sauerstoffatom bedeutet, sowie deren Säureadditionssalze.

4. N-(2-substituierte-4-Pyridyl)harnstoffe, nach Anspruch 1, dadurch gekennzeichnet, dass jeweils $R_1$ ein Wasserstoffatom, Y ein Sauerstoffatom und $R_2$ eine mit einem Halogenatom oder einer Methylgruppe substituierte Phenylgruppe bedeutet, sowie deren Säureadditionssalze.

5. N-(2-substituierte-4-Pyridyl)-N'-phenylharnstoffe und deren Säureadditionssalze gemäss Anspruch 1.

6. N-(2,6-Dichlor-4-pyridyl)-N'-(substituierte phenyl)harnstoffe, sowie deren Säureadditionssalze gemäss Anspruch 1.

7. N-(2-Trifluormethyl-4-pyridyl)-N'-(3-fluorphenyl)harnstoff sowie dessen Säureadditionssalze gemäss Anspruch 1.

8. N-(2-Fluor-4-pyridyl)-N'-phenylharnstoff sowie dessen Säureadditionssalze gemäss Anspruch 1.

9. N-(2-Methoxy-4-pyridyl)-N'-phenylharnstoff sowie dessen Säureadditionssalze gemäss Anspruch 1.

10. N-(2-Brom-4-pyridyl)-N'-phenylharnstoff sowie dessen Säureadditionssalze gemäss Anspruch 1.

11. N-(2-Trifluormethyl-4-pyridyl)-N'-phenylharnstoff sowie dessen Säureadditionssalze gemäss Anspruch 1.

12. Verfahren zur Herstellung von N-(2-substituierten-4-Pyridyl)harnstoffen nach Anspruch 1, dadurch gekennzeichnet, dass man

a) ein entsprechendes 2-substituiertes-4-Aminopyridin mit einem entsprechenden Phenylisocyanat umsetzt oder
b) ein entsprechendes 2-substituiertes-4-Pyridylisocyanat, bei dem X keine $NH_2$- oder OH-Gruppe ist, mit einem entsprechenden Anilin umsetzt oder
c) ein entsprechendes 2-substituiertes-Isonikotinoylazid, bei dem X keine $NH_2$- oder OH-Gruppe ist, mit einem entsprechenden Anilin umsetzt.

13. Pflanzenwachstumsregulatoren, dadurch gekennzeichnet, dass sie als Wirkstoff mindestens einen N-(2-substituierten-4-Pyridyl)harnstoff nach Anspruch 1 enthalten.

## Claims

1. N-(2-substituted-4-pyridyl)ureas of the general formula I

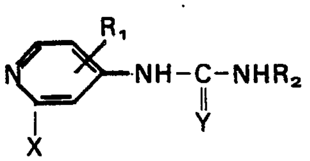

(I)

wherein X represents a halogen atom, a hydroxy, lower alkoxy, lower alkylthio, lower alkoxycarbonyl, amino, lower alkylcarbonylamino, benzoylamino, lower alkoxycarbonylamino, cyano, or trifluoromethyl group, wherein the lower alkyl or alkoxy group shall be lower than $C_6$, $R_1$ represents a hydrogen atom, a halogen atom or lower alkyl group, $R_2$ represents an aromatic group, optionally substituted by a lower alkyl or alkoxy, hydroxy, trifluoromethyl group or a halogen atom, and the acid addition salts thereof.

2. N-(2-substituted-4-pyridyl)ureas according to claim 1 characterized in that $R_1$ represents a hydrogen atom, and the acid addition salts thereof.

3. N-(2-substituted-4-pyridyl)ureas characterized in that Y represents an oxygen atom and the acid addition salts thereof.

4. N-(2-substituted-4-pyridyl)ureas characterized in that $R_1$ represents a hydrogen atom, Y represents an oxygen atom and $R_2$ represents a phenyl group substituted by a halogen atom or a methyl group and the acid addition salts thereof.

5. N-(2-substituted-4-pyridyl)-N'-phenylureas and the acid addition salts thereof according to claim 1.

6. N-(2,6-dichloro-4-pyridyl)-N'-(substituted-phenyl)ureas and the acid addition salts thereof according to claim 1.

7. N-(2-trifluoromethyl-4-pyridyl)-N'-(3-fluorophenyl)urea and the acid addition salts thereof according to claim 1.

8. N-(2-fluoro-4-pyridyl)-N'-phenylurea and the acid addition salts thereof according to claim 1.

9. N-(2-methoxy-4-pyridyl)-N'-phenylurea and the acid addition salts thereof according to claim 1.

10. N-(2-bromo-4-pyridyl)-N'-phenylurea and the acid addition salts thereof according to claim 1.

11. N-(2-trifluoromethyl-4-pyridyl)-N'-phenylurea and the acid addition salts thereof according to claim 1.

12. Method for preparing N-(2-substituted-4-pyridyl) ureas according to claim 1, characterized in that

a) a corresponding 2-substituted-4-aminopyridin is reacted with a corresponding phenylisocyanate, or
b) a corresponding 2-substituted-4-pyridylisocyanate wherein X is not a $NH_2$- or OH-group, is reacted with a corresponding aniline, or
c) a corresponding 2-substituted-isonicotinoylazide, wherein X is not a $NH_2$- or OH-group, is reacted with a corresponding aniline.

13. Plant growth regulators, characterized in that they contain at least one N-(2-substituted-4-pyridyl)urea according to claim 1.

## Revendications

1. Une N-(substituée-2-pyridyl-4)urée représentée par la formule générale (I):

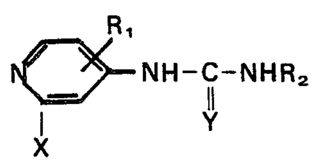

(I)

dans laquelle

X représente un atome d'halogène, un groupe hydroxy, un groupe alcoxy inférieur, un groupe alcoylthio inférieur, un groupe alcoxycarbonyle inférieur, un groupe amino, un groupe alcoylcarbonylamino inférieur, un groupe benzoylamino, un groupe alcoxycarbonylamino inférieur, un groupe cyano ou un groupe trifluorométhyle dans lesquels le groupe alcoyle ou alcoxy inférieur doit être inférieur à $C_6$,

$R_1$ représente un atome d'hydrogène, un atome d'halogène ou un groupe alcoyle inférieur,

$R_2$ représente un atome aromatique substitué le cas échéant par un groupe alcoyle ou alcoxy inférieur, un groupe hydroxy, un groupe trifluorométhyle ou un atome d'halogène, et

Y représente un atome d'oxygène ou de soufre, X devant être uniquement un atome de chlore lorsque $R_1$ est un atome d'halogène,
et ses sels d'addition avec un acide.

2. Une N-(substituée-2-pyridyl-4)urée selon la revendication 1, caractérisée en ce que $R_1$ est un atome d'hydrogène, et ses sels d'addition avec un acide.

3. Une N-(substituée-2-pyridyl-4)urée selon la revendication 1, caractérisée en ce que Y est un atome d'oxygène, et ses sels d'addition avec un acide.

4. Une N-(substituée-2-pyridyl-4)urée selon la revendication 1, caractérisée en ce que respectivement, $R_1$ représente un atome d'hydrogène, Y représente un atome d'oxygène et $R_2$ représente un groupe phényle substitué par un atome d'halogène ou un groupe méthyle, et ses sels d'addition avec un acide.

5. Une N-(substituée-2-pyridyl-4)-N'-phénylurée et ses sels d'addition avec un acide selon la revendication 1.

6. Une N-(dichloro-2,6-pyridyl-4)-N'-(phényl-substituée)urée et ses sels d'addition avec un acide selon la revendication 1.

7. Une N-(trifluorométhyl-2-pyridyl-4)-N'-(fluoro-3-phényl)urée et ses sels d'addition avec un acide selon la revendication 1.

8. Une N-(fluoro-2-pyridyl-4)-N'-phénylurée et ses sels d'addition avec un acide selon la revendication 1.

9. Une N-(méthoxy-2-pyridyl-4)-N'-phénylurée et ses sels d'addition avec un acide selon la revendication 1.

10. Une N-(bromo-2-pyridyl-4)-N'-phénylurée et ses sels d'addition avec un acide selon la revendication 1.

11. Une N-(trifluorométhyl-2-pyridyl-4)-N'-phénylurée et ses sels d'addition avec un acide selon la revendication 1.

12. Un procédé de production de N-(substitué-2-pyridyl-4)urée selon la revendication 1 caractérisé en ce que

a) on fait réagir une substituée-2-amino-4-pyridine adéquate avec un phénylisocyanate adéquat, ou

b) on fait réagir un substitué-2-pyridyl-4-isocyanate adéquat, dans lequel X n'est ni un groupe $NH_2$ ni un groupe OH, avec une aniline adéquate, ou

c) on fait réagir un substitué-2-isonicotinoylazide adéquat, dans lequel X n'est ni un groupe $NH_2$ ni un groupe OH, avec une aniline adéquate.

13. Des régulateurs de croissance des plantes, caractérisés en ce qu'ils contiennent comme composé actif au moins une N-(substituée-2-pyridyl-4)urée selon la revendication 1.